**Europäisches Patentamt**

**European Patent Office**

**Office européen des brevets**

(19)

(11) **EP 1 285 904 A1**

(12) **EUROPEAN PATENT APPLICATION**

| | |
|---|---|
| (43) Date of publication:<br>**26.02.2003 Bulletin 2003/09** | (51) Int Cl.⁷: **C07C 19/08**, C07C 21/18,<br>C07C 43/00, C07C 43/17,<br>C07C 17/087, C07C 17/363 |
| (21) Application number: **01116701.2** | |
| (22) Date of filing: **17.07.2001** | |

| | |
|---|---|
| (84) Designated Contracting States:<br>**AT BE CH CY DE DK ES FI FR GB GR IE IT LI LU MC NL PT SE TR**<br>Designated Extension States:<br>**AL LT LV MK RO SI** | • **Lekontseva, Galina Ivanovna**<br>**Perm (RU)** |
| | (74) Representative:<br>**Held, Stephan, Dr.rer.nat., Dipl.-Chem. et al Patentanwälte,**<br>**Hagemann, Braun und Held,**<br>**Postfach 86 03 29**<br>**81630 München (DE)** |
| (71) Applicant: **Igumnov, Sergei Mikhailovich**<br>**1445-363 Moscow (RU)** | |
| (72) Inventors:<br>• **Igumnov, Sergei Mikhailovich**<br>**Moscow (RU)** | |

(54) **Process for producing fluorinated aliphatic compounds**

(57)     The present invention relates to a process for producing fluorinated aliphatic compounds by pyrolysis of perfluoro-carboxylic acids and their derivatives - halides and esters. The pyrolysis is carried out in the presence of a catalyst consisting of a carrier most preferably selected from the series comprising active carbon, magnesium oxide, calcium oxide, barium oxide, zinc oxide, aluminum oxide, nickel oxide, oxides of silicon promoted with alkali metal halides, selected from the series comprising fluorides, chlorides bromides, iodides of sodium, potassium, rubidium, cesium, at a temperature in the range of from about 100°C to about 450°C; with producing fluorinated, aliphatic compounds selected from the series comprising perfluoroolefins, polyflubroolefins and their derivatives; and also in the presence additionally of hydrogen fluoride with the formation of fluorinated aliphatic compounds selected from the series comprising polyfluoroalkanes and their derivatives.

**EP 1 285 904 A1**

**Description**

Field of the Art

The present invention relates to the fluoroorganic chemistry, particularly to a process for producing fluorinated aliphatic compounds by pyrolysis of perfluoro- and polyfluorocarboxylic acids and their derivatives - halides and esters. The products of the pyrolysis, depending on the conditions under which it is carried out, are fluorinated olefins, perfluoroalkylvinyl ethers and polyfluoroalkanes. Fluorinated olefins and perfluoroalkylvinyl ethers are used as starting materials for obtaining polymer materials with improved operational characteristics, lubricating oils, elastomers, ion-exchange membranes for the electrolysis of aqueous solutions of alkali metal halides, etc. Polyfluoroalkanes, owing to their chemical inertness and thermal stability, find application as components of mix coolants, actuating fluids of thermocompressors, porophores in manufacturing foamed plastics and polyurethane foams, gas dielectrics, propellants, inert solvents, reagents for dry etching in manufacturing integrated circuits, and also in formulations of fire-extinguishing means.

At present a necessity is felt in the provision of an industrial process for producing fluorinated olefins, polyfluoroalkanes, perfluoroalkylvinyl ethers from different available organofluorine compounds under relatively mild reaction conditions with a high yield.

Prior Art

It is known that polyfluoroalkanes, particularly such as promising ozone-safe Freons 125, 227ea, are produced mainly by the hydrofluorination of perfluoroolefins - tetrafluoroethylene (TFE) and hexafluoropropylene (HFP), the production of which involves definite difficulties. TFE is explosion-hazardous, highly toxic, and self-polymerizes. TFE and HFP are produced by the high-temperature pyrolysis of chlorine- and fluorine-containing hydrocarbons, whose production in accordance with the decision of the Montreal Proto-. col concerning substances destroying the ozone layer is reduced because they deteriorate considerably the ecological situation. Therefore the possibility of obtaining polyfluoroalkanes (Freon 125, 227ea and the like) and olefins as such (TFE, HFP) from other available starting materials, namely, perfluoro- and polyfluorocarboxylic acids and their derivatives by a more simple process is a very urgent problem. The claimed process solves this problem.

It is known that in the pyrolysis of esters of perfluoroalkoxycarboxylic acids of the formula

$$ICF_2CF_2OQCF(CF_3)COOR,$$

where

Q is $OCF_2CF(CF_3)$ $[OCF(CF_3)CF_2]_m$, $[OCF(CF_3)CF_2]_n$;
m is 0 to 7;
n is 1 to 4;
R is $(C_1-C_6)$ alkyl

vinyl ethers are formed above a layer of carbonate, phosphate, sulfite, sulfate of an alkali or alkali-earth metal (US 4594458, C07C 43/16, publ. 10.06.86). In this process, from an ester first a salt is obtained by treating $NaHCO_3$ in a methanol solution, and then the salt is subjected to pyrolysis at 140-260°C. In accordance with this process, perfluoro (8-iodo-4-methyl-3,6-dioxaoctene-1) is obtained with a yield of 71.8%.

A disadvantage of this process is the necessity of carrying out the intermediate step of obtaining an alkali metal salt and the necessity of drying this salt thoroughly, because the presence of moisture leads to the formation of byproducts. Furthermore, toxic, fire- and explosion-hazardous methanol is used for carrying out the reaction.

In the pyrolysis of ethyl esters of pentafluoropropionic and heptafluorobutyric acid at 350-413°C and a pressure of about 8-80 gPa tetrafluoroethylene and hexafluoropropylene are formed, respectively, with a yield of about 30%, as well as an insignificant amount of pentafluorethane and heptafluoropropane (Int. J. Chem. Kinet., 1982, 14, No. 3, 291).

Known in the art is pyrolysis of polyfluoroalkoxyperfluoropropionic acid fluorides on sodium carbonate at 200-220°C, which gives polyfluoroalkylperfluorovinyl ethers with a yield of 85-91% (Zhurnal Organicheskoi Khimii, 1978, 14, No. 3, 487). The pyrolysis proceeds in accordance with the following scheme:

$$R_fCF(CF_3)OCF(CF_3)COF \rightarrow R_fCF(CF_3)OCF=CF_2,$$

where $R_f$ is $CF_3CF_2$, $CF_3(CF_2)_3$, $ClCF_2CF_2$.

$Na_2CO_3$ is a reagent which is consumed in the process of the synthesis, forming NaF.

In the pyrolysis of polyfluorocarboxylic acid halides of the formula $XC_nF_{2n}COl$ (wherein X is H, F; n > 1; l is a haloid) at 200-500°C, fluorolefins of the formula $XC_nF_{2n-1}$ are formed (US 3020321, 260-653.3, publ. 06.02.62). The reaction is carried out with oxides of Group IIA metals and silicon or with oxygen-containing salts of Group IA and Group IIA metals of the Periodic System.

The disadvantages of this process are a high reaction temperature (mainly 380°C) and direct participation of oxides and oxygen-containing salts in the process with the formation of inorganic fluorides, which makes the process unstable.

Decarbonylation of polyfluorocarboxylic acid fluorides is known in the art, which leads to obtaining polyfluoroalkanes in accordance with the following scheme:

$$H(CF_2)_nCOF \rightarrow H(CF_2)_nF.$$

The reaction proceeds in the presence of a catalyst: anhydrous aluminum oxide (RU 659555, C07C 19/08, publ. 30.04.79) or under heating acyl fluoride with antimony pentafluoride (US 3555100, C07C 19/08, publ. 12.01.71). As a result, 1-hydroperfluorohexane was obtained with a yield of 87-93%.

The prior-art processes cited above depend on starting materias, because monohydroperfluoroalkanes can be produced only from corresponding monohydroperfluorocarboxylic acid fluorides, which are not always available. They do not allow selective introduction of hydrogen into the carbon chain either.

High-temperature decarboxylation (620°C and 120 mm Hg) of 2-hydroperfluorobutyric acid gives 2-hydropentafluoropropylene with the yield of 92% (2-Hydropentafluoro propylene. Khim. Promyshl. Ser. Prikladnaya Khimiya, Moscow, NIITEK-hiM, 1979, pp. 1-2). The reaction proceeds in accordance with the following scheme:

$$CF_3CHFCF_2COOH \rightarrow CF_3CH=CF_2.$$

Decarboxylation of $\alpha$- hydrohexafluoroisobutyric acid in dimethyl formamide gives 2,2-dihydrohexafluoropropane with the yield of 65% (Izv. AN SSSR, Ser. Khimiya, 1977, No. 5, 1112).

The reaction proceeds in accordance with the following scheme:

$$(CF_3)_2CHCOOH \rightarrow CF_3CH_2CF_3.$$

With the help of this process only dihydroperfluoroalkanes can be produced.

It is known that decarboxylation of fluorocarboxylic acids of the formula $R(CFR^1)_n(CFR^2)_mOCF(CF_2X)COOH$, where R is $SO_2Z$, $POZ_2$, COZ; Z is $OR^3$, F, Cl, Br, I; $R^3$ is alkyl or aryl; $R^1$ and $R^2$ each are F, Cl, perfluoro- or chlorofluoroalkyl; X is Cl, Br, I; n is from 0 to 3; m is from 0 to 3, in the presence of an activator $Na_2CO_3$, ZnO or $SiO_2$ in an organic solvent (mono-, di- or tetraglyme) at 50-150°C makes it possible to obtain corresponding vinyl ethers of the following formula: $R(CFR^1)_n(CFR^2)_mOCF=CF_2$ (US 4358412, C07F 9/113, publ. 09.11.82).

Disclosure of the Invention

It is an object of the present invention to provide a universal industrial process for producing fluorinated aliphatic compounds with a high yield from available starting materials.

Another object of the present invention is to provide a process for producing fluorinated aliphatic compounds that are -fluorinated olefins and perfluoroalkylvinyl ethers, by pyrolysis of perfluoro- and polyfluorocarboxylic acids, their halides and esters.

Still another object of the present invention is to provide a process for producing fluorinated aliphatic compounds that are polyfluoroalkanes, by pyrolysis of perfluoro- and polyfluorocarboxylic acids, their halides and esters in the presence of hydrogen fluoride.

Said objects are accomplished by the present invention, wherein a process for producing fluorinated aliphatic compounds is disclosed, consisting in carrying out pyrolysis of perfluoro- and polyfluorocarboxylic acids and their derivatives selected from the acid halides and esters, on a catalyst consisting of a carrier, most preferably selected from the series comprising active carbon, magnesium oxide, calcium oxide, barium oxide, zinc oxide, aluminum oxide, nickel oxide, oxides of silicon promoted with alkali metal halides at a temperature of 100-450°C, optionally in the presence of hydrogen fluoride.

Carrying out pyrolysis in the absence of hydrogen fluoride leads to the formation of fluorinated olefins and perfluoroalkylvinyl ethers. In the case of pyrolysis carried out in the presence of hydrogen fluoride polyfluoroalkanes are obtained as the target end products.

Best Variants of Carrying out the Invention

The process of pyrolysis is carried out continuously in a flow-through system, for which purpose a tubular reactor is used. The reactor is provided with electric heating, with a thermocouple sheath, with pipes for feeding the starting components and discharging the reaction products. The factor of filling the reactor with a catalyst is up to 0.8.

The catalyst consists of a carrier preferably selected from the series comprising active carbon, silicon oxides, oxides of metals of Groups II, III, IV of the Periodic System, oxides of transition metals promoted with alkali metal halides. Compounds selected from the series comprising fluorides, chlorides, bromides, iodides of sodium, potassium, rubidium, cesium are used as said metal halides. It is most preferable to use magnesium oxide, calcium oxide, barium oxide, zinc oxide, aluminum oxide, nickel oxide as the above-indicated metal oxides. Examples of such catalysts can be $KF/SiO_2$ $NaF/SiO_2$, $CsF/NiO$, $NaF/Al_2O_3$, $KF/CaO$, $CsF/SiO_2$, $NaCl/C_{act.}$, $KF/MgO$, $KI/Al_2O_3$, etc. Active carbon and silicon dioxide, promoted with potassium fluoride, are the most preferable catalysts.

Optimal content of halides in the catalyst is from 20 to 50 wt.%. A reduction of the halide concentration to less than 20 wt.% leads to reducing the yield of the target product. An increase of the halide concentration above 50 wt.% does not influence substantially the process and is, therefore, inexpedient.

For preparing the catalyst, the carrier is mixed with an aqueous solution of an alkali metal halide, kept for up to 24 hours at room temperature, and then dried in a drying cabinet at 180-210°C to constant weight.

For creating a reaction zone, the prepared catalyst is charged into the reactor and heated in a stream of dry nitrogen, with the temperature increased gradually from 180 to 350°C for four hours. Then the starting reagent is fed into said reaction zone, said starting reagent being selected from the series comprising perfluorocarboxylic acids, polyfluorocarboxylic acids of a normal or iso-structure, optionally containing a haloid other than fluorine, or their derivatives, such as acid halides or esters. Examples of said starting reagent can be perfluoropropionic acid, perfluorobutyric acid, perfluorovaleric acid, perfluoropelargonic acid, ω-hydroperfluorobutyric acid, ω-hydroperfluorovaleric acid, perfluoroisobutyric acid, perfluoropropionic acid chloride, perfluoropropionic acid fluoride, perfluoropropionic acid bromide, perfluoroisobutyric acid fluoride, perfluorobutyric acid chloride, perfluorovaleric acid fluoride, perfluorovaleric acid chloride, perfluoroenanthic acid chloride, perfluoropelargonic acid fluoride, ω-hydroperfluorovaleric acid fluoride, ω-hydroperfluoropropionic acid fluoride, perfluoropropoxyisopropionic acid fluoride, perfluoropropoxyisopropionic acid chloride, perfluoromethoxyisopropionic acid fluoride, 2-bromoperfluoroethoxyisopropionic acid fluoride, methyl ester of perfluoropropionic acid, ethyl ester of perfluoropropionic acid, methyl ester of perfluoroisobutyric acid, ethyl ester of perfluorovaleric acid, ethyl ester of perfluoroenanthic acid, methyl ester of perfluoropelargonic acid, methyl ester of ω-hydroperfluorovaleric acid, ethyl ester of perfluoropropoxyisopropionic acid,- methyl ester of perfluoropropoxyisopropionic acid, methyl ester of 2-bromoperfluoroethoxyisopropionic acid, methyl ester of perfluoropentoxyisopropionic acid.

The process of pyrolysis of the starting reagent in the presence of a catalyst is carried out in a sufficiently wide temperature range of 100 to 450°C. At a temperature below 100°C the reaction slows down to such an extent that the conversion of the starting compounds does not exceed 5-10%. A temperature increase to 450°C causes destruction of the starting components and of the target products. For obtaining fluorinated olefins and perfluoroalkylvinyl ethers the temperature of 170-250°C is preferable. The synthesis of polyfluoroalkanes should be carried out preferably at a temperature of 250-350°C.

The above-described catalytic pyrolysis of perfluorocarboxylic acids, polyfluorocarboxylic acids and their derivatives gives fluorinated olefins and perfluoroalkylvinyl ethers, such as, e.g., tetrafluoroethylene,. hexafluoropropylene, perfluoro-1-butene, perfluoro-2-butene, perfluoro-2-pentene, perfluoro-2-hexene, perfluoro-2-octene, perfluoro-3-octene, perfluoro-4-octene, 1-hydroperfluoro-2-butene, trifluoroethylene, perfluoropropylvinyl ether, perfluoromethylvinyl ether, perfluoropentylvinyl ether, 2-bromoperfluoroethylvinyl ether.

In the case the above-described catalytic pyrolysis of perfluorocarboxylic acids, polyfluorocarboxylic acids and their derivatives is carried out with introducing hydrogen fluoride into the reaction zone, there takes place hydrofluorination of the fluorinated olefins and perfluoroalkylvinyl ethers which form as a result of the pyrolysis, and the obtained final products are polyfluoroalkanes containing at least one hydrogen atom, and ethers, for instance, 1-hydropentafluoroethane, 2-hydroheptafluoropropane, 2-hydroperfluorobutane, 1,3-dihydroperfluorobutane, 2-hydroperfluoroethylpropyl ether, 1-bromo-4-hydroperfluorodiethyl ether.

When producing polyfluoroalkanes, a 1.5-2.0-fold molar excess of hydrogen fluoride to the starting reagent is optimal. Feeding a smaller amount of hydrogen fluoride leads to an appreciable lowering of the yield of the target products, whereas an increase in the amount of the fed hydrogen fluoride is inexpedient.

Hydrogen fluoride can be introduced into the reaction zone simultaneously with the starting reagent or after performing the pyrolysis step. In this case hydrogen fluoride is fed to the reaction zone through the pipe into the middle part of the reactor.

The products of pyrolysis, i.e., fluorinated olefins and perfluoroalkylvinyl ethers, are condensed in a cooled collecting tank.

When producing polyfluoroalkanes, there remains unreacted hydrogen fluoride. To remove it, acid products of the pyrolysis are first washed with an alkali solution, then neutralized additionally on a column with a lime chemical absorber, and after that condensed at a temperature of -30 to -50°C. The target products are isolated from the condensate by rectification and then identified by IR and NMR spectroscopy techniques.

In accordance with the present process there have been produced, in particular, 1-hydropentafluoroethane (Freon 125), 2-hydroheptafluoropropane (Freon 227ea) with the yield of 98%, and other polyfluoroalkanes, whose yield was not lower than 85%. In accordance with the present process there have been also produced various perfluoroolefins, polyfluoroolefins and perfluoroalkylvinyl ethers with a yield of up to 95%.

Examples

The Examples which follow are presented for illustrating the invention but for limiting it.

Example 1

Synthesis of Perfluoro-2-butene

A 0.3 dm$^3$-capacity tubular reactor made from stainless steel, provided with electric heating, with a thermocouple sheath, with pipes for feeding the starting components and discharging the reaction products, is charged with about 0.23 dm$^3$ of a catalyst which -is silicon dioxide promoted with potassium fluoride in an amount of 35 wt.%. The catalyst is heated in a stream of dry nitrogen with a gradual temperature increase from 180 to 350°C for four hours. Then the temperature is lowered to 240°C, and 40 g of methyl ester of perfluorovaleric acid are fed with the rate of 20 g/hour. The gas mixture outgoing from the reactor is condensed in a trap cooled down to -30°C, and subjected to low-temperature rectification, as a result of which 27.4 g of perfluoro-2-butene are obtained.. The yield of the target product is 95.1%.

Example 2

Synthesis of 2-Hydroheptafluoropropane

A tubular reactor described in Example 1 is charged with 0.23 dm$^3$ of a catalyst which is active carbon promoted with potassium fluoride in an amount of 30 wt.%. The catalyst is heated in a stream of dry nitrogen with a gradual temperature increase from 180 to 350°C for four hours. Then the temperature is lowered to 250°C, and 50 g of methyl ester of perfluoroisobutyric acid and 7.5 g of hydrogen fluoride are fed during 1 hour.

The gas mixture outgoing from the reactor is passed through a solution of potassium hydroxide, collected in a trap cooled down to -30°C, and subjected to low-temperature rectification. 36.5 g of the target product are obtained, this corresponding to the yield of 98.0%.

Example 3

Synthesis of 1-Hydropentafluoroethane

A tubular reactor described in Example 1 is charged with about 0.21 dm$^3$ of a catalyst which is silicon dioxide promoted with cession fluoride in an amount of 40 wt.%. The catalyst is heated in a stream of dry nitrogen with a gradual temperature increase from 180 to 350°C for four hours.

Then the temperature is lowered to 260°C, and 20 g of perfluoropropionic acid fluoride and 4 g of hydrogen fluoride are fed during 1 hour, the hydrogen fluoride being fed into the middle part of the reactor.

The gas mixture outgoing from the reactor is condensed in a trap cooled down to -30°C, and subjected to low-temperature rectification. 13 g of the target product indicated in the heading of this Example are obtained, this corresponding to the yield of 89.7%.

The subsequent syntheses (Examples 4-19) are carried out similarly to Examples 1, 2. The synthesis conditions and results are presented in the Table.

Thus, a unique process has been developed for the synthesis of fluorinated aliphatic compounds by a catalytic pyrolysis of different starting reagents: perfluorocarboxylic acids, polyfluorocarboxylic acids and their derivatives. The present process makes it possible to produce perfluoroolefins, polyfluoroolefins and perfluoroalkylvinyl ethers with a yield of up to 95%, as well as to produce polyfluoroalkanes with a yield of up to 98% with a selective introduction of hydrogen into the carbon chain.

EP 1 285 904 A1

**Pyrolysis conditions and resutls**

Table

| Nos. | Starting compound | | Catalyst | | Temp., °C | Metering rate, g/h | | Duration, h | Resulting compound | | Amount of product, g | Yield, % |
|---|---|---|---|---|---|---|---|---|---|---|---|---|
| | | | Composition | Content of promotor, wt.% | | Starting Comp-d | HF | | | | | |
| 4 | Methyl ester of per-fluoroisobutyric acid | $CF_3$ <br> $\|$ <br> $CF_3 - CF\text{-}COOCH_3$ | $KF/SiO_2$ | 30 | 300 | 10 | | 3.0 | Hexafluoropropylene | $CF_3CF{=}CF_2$ | 18.0 | 91.8 |
| 5 | Perfluorovaleric acid | $CF_3(CF_2)_3COOH$ | $KF/SiO_2$ | 30 | 270 | 20 | | 2.0 | Perfluoro- 1,2-butenes | $CF_3CF{=}CFCF_3$ <br> $CF_3CF_2CF{=}CF_2$ | 27.6 | 91.1 |
| 6 | Perfluoropropoxyiso-propionic acid fluoride | $CF_3CF_2CF_2OCFCOF$ <br> $\|$ <br> $CF_3$ | $CsF/NiO$ | 20 | 170 | 16 | | 2,0 | Perfluoropropylvinyl ether | $CF_3CF_2\ CF_2OCF{=}CF_2$ | 23.0 | 89.8 |
| 7 | Ethyl ester of per-fluoropropoxyisopro-pionic acid | $CF_3CF_2CF_2OCFCOOC_2H_5$ <br> $\|$ <br> $CF_3$ | $NaF/Al_2O_3$ | 30 | 300 | 14 | | 2.0 | Perfluoropropylvinil ether | $CF_3CF_2\ CF_2OCF{=}CF_2$ | 13.2 | 60.6 |
| 8 | 2-Bromoperfluoroeth-oxyisopropionic acid fluoride | $BrCF_2CF_2OCFCOF$ <br> $\|$ <br> $CF_3$ | $KF/CaO$ | 20 | 200 | 20 | | 1.0 | Perfluoro-2-bromoethylvinyl ether | $BrCF_2CF_2OCF{=}CF_2$ | 15.4 | 95.1 |
| 9 | Ethyl ester of perfluoropropionic acid | $CF_3CF_2COOC_2H_5$ | $CsF/SiO_2$ | 40 | 280 | 20 | 4.0 | 1.5 | 1-Hydropenta-fluoroethane | $CF_3CF_2H$ | 17.6 | 94.2 |
| 10 | Perfluoroisobutyric asid fluoride | $CF_3 - CF\text{-}COF$ <br> $\|$ <br> $CF_3$ | $NaCl/C_{akt.}$ | 35 | 300 | 15 | 2.5 | 2.0 | 2-Hydropenta-fluoropropane | $CF_3CFHCF_3$ | 21.4 | 90.5 |
| 11 | ω-Hydroperfluorova-leric acid | $H(CF_2)_4COOH$ | $KF/MgO$ | 25 | 130 | 20 | 3.0 | 2.0 | 1,3-Dihydroper fluorobutane | $HCF_2CF_2CFHCF_3$ | 28.0 | 85.3 |
| 12 | Perfluorovaleric acid | $CF_3(CF_2)_3COOH$ | $KI/Al_2O_3$ | 30 | 160 | 20 | 2.7 | 1..5 | 2-Hydroper-fluorobutane | $CF_3CF_2CFHCF_3$ | 21.2 | 85.0 |
| 13 | Perflupropropionic acid fluoride | $CF_3CF_2COF$ | $KF/C_{act.}$ | 30 | 260 | 15 | | 2.0 | Tetrafluoro-ethylene | $CF_2{=}CF_2$ | 15.8 | 87.5 |
| 14 | Perfluoropropionic acid chloride | $CF_3CF_2COCl$ | $KF/C_{act.}$ | 20 | 290 | 20 | 3.7 | 3.0 | 1-Hydropenta-fluoroethane | $CF_3CF_2H$ | 36.6 | 92.5 |

| | | | | | | | | | | | | | |
|---|---|---|---|---|---|---|---|---|---|---|---|---|---|
| 15 | ω-Hydroperfluoropro-pyonic acid fluoride | HCF₂CF₂COF | KF/Cₐct. | 30 | 200 | 20.0 | – | 1.0 | Trifluoroeth-ylene | CF₂=CFH | 9.8 | 88.3 |
| 16 | Methyl ester of per-fluoromethoxyisopro-pyoric acid | CF₃OCFCOOCH₃ \| CF₃ | KF/SiO₂ | 25 | 220 | 15.0 | 2.0 | 2.0 | Perfluorometh-ylvinyl ether | CF₃OCF=CF₂ | 20.4 | 93.2 |
| 17 | Methyl ester of per-fluoropropionic acid | CF₃CF₂COOCH₃ | KF/Cₐct. | 30 | 270 | 16 | 3.0 | 2.0 | 1-Hydropenta-fluoroethane | CF₃CF₂H | 21.2 | 98.1 |
| 18 | Methyl ester of per-fluoroisobutyric acid | CF₃ \| CF₃-CF-COOHCH₃ | NaF/Cₐct. | 50 | 300 | 30 | 5.1 | 2.0 | 2-Hydrohepta-fluoropropane | CF₃CFHCF₃ | 41.2 | 92.0 |
| 19 | Methyl ester of per-fluorovaleric acid | CF₃CF₂CF₂COOCH₃ | KF/SiO₂ | 20 | 250 | 25 | 3.5 | 2.0 | 2-Hydroper-fluorobutane | CF₃CF₂CFHCF₃ | 45.1 | 93.5 |

8

**Claims**

1. A process for producing fluorinated aliphatic compounds, comprising the following steps:

    introducing a catalyst consisting of a carrier promoted with alkali metal halides into a reactor;
    creating a reaction zone;
    maintaining a temperature ranging from about 100°C to about 450°C in said zone;
    introducing into said zone a starting reagent selected from the series comprising perfluorocarboxylic acids, polyfluorocarboxylic acids and their derivatives, and contacting thereof with said catalyst;
    pyrolysis of said starting reagent in the presence of said catalyst in said reaction zone to obtain fluorinated aliphatic compounds that are an unsaturated compound selected from the series comprising perfluoroolefins, polyfluoroolefins and their derivatives.

2. A process according to claim 1, wherein compounds selected from the series comprising active carbon, silicon oxides, oxides of metals of Groups II, III, IV of the Periodic System, transition metal oxides are used as said carrier.

3. A process according to claim 2, wherein compounds selected from the series comprising magnesium oxide, calcium oxide, barium oxide, zinc oxide, aluminum oxide, nickel oxide are used as said metal oxides.

4. A process according to claim 1, wherein compounds selected from the series comprising fluorides, chlorides, bromides, iodides of sodium, potassium, rubidium, cesium are used as said alkali metal halides.

5. A process according to claim 1, wherein the content of said alkali metal halides in said catalyst is within a range of from about 20 to about 50%.

6. A process for producing fluorinated aliphatic compounds, comprising the following steps:

    introducing a catalyst consisting of a carrier promoted with alkali metal halides into a reactor;
    creating a reaction zone;
    maintaining a temperature pranging from about 100°C to about 450°C in said zone;
    introducing into said zone a starting reagent selected from the series comprising perfluorocarboxylic acids, polyfluorocarboxylic acids and their derivatives, and contacting thereof with said catalyst;
    pyrolysis of said starting reagent in the presence of said catalyst in said reaction zone to obtain fluorinated aliphatic compounds that are an unsaturated compound selected from the series comprising perfluoroolefins, polyfluoroolefins and their derivatives;
    introducing hydrogen fluoride into said zone, as a result of which there takes place hydrofluorination of said unsaturated compound with the formation of fluorinated aliphatic compounds that are an unsaturated compound selected from the series comprising polyfluoroalkanes and their derivatives.

7. A process according to claim 6, wherein said hydrogen fluoride is introduced into said zone simultaneously with said starting reagent.

8. A process according to claim 6, wherein said hydrogen fluoride is introduced into said zone after said step of pyrolysis, as a result of which the formation of said unsaturated compound takes place.

9. A process according to claim 6, wherein compounds selected from the series comprising active carbon, silicon oxides, oxides of metals of Groups II, III, IV of the Periodic System, transition metal oxides are used as the carrier.

10. A process according to claim 9, wherein compounds selected from the series comprising magnesium oxide, calcium oxide, barium oxide, zinc oxide, aluminum oxide, nickel oxide are used as said metal oxides.

11. A process according to claim 6, wherein compounds selected from the series comprising fluorides, chlorides, bromides, iodides of sodium, potassium, rubidium, cesium are used as said alkali metal halides.

12. A process according to claim 6, wherein the content of said alkali metal halides in said catalyst is within a range of from about 20 to about 50%.

13. A process according to claim 6, wherein said hydrogen fluoride is used in an amount of 1.5-2 moles per mole of

said starting reagent.

**European Patent Office**

## EUROPEAN SEARCH REPORT

Application Number

EP 01 11 6701

| Category | Citation of document with indication, where appropriate, of relevant passages | Relevant to claim | CLASSIFICATION OF THE APPLICATION (Int.Cl.7) |
|---|---|---|---|
| Y | SU 539 863 A (PLASHKIN V S)<br>25 December 1976 (1976-12-25)<br>* abstract * | 1-13 | C07C19/08<br>C07C21/18<br>C07C43/00<br>C07C43/17 |
| Y | RU 2 134 257 C (KLADNAJA KHIM;PERM ROSSIJSKOGO NAUCHNOGO TSE)<br>10 August 1999 (1999-08-10)<br>* the whole document * | 1-13 | C07C17/087<br>C07C17/363 |

**TECHNICAL FIELDS SEARCHED (Int.Cl.7)**

C07C

The present search report has been drawn up for all claims

| Place of search | Date of completion of the search | Examiner |
|---|---|---|
| MUNICH | 18 December 2002 | Seelmann, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03 82 (P04C01)

**ANNEX TO THE EUROPEAN SEARCH REPORT
ON EUROPEAN PATENT APPLICATION NO.**

EP 01 11 6701

This annex lists the patent family members relating to the patent documents cited in the above-mentioned European search report.
The members are as contained in the European Patent Office EDP file on
The European Patent Office is in no way liable for these particulars which are merely given for the purpose of information.

18-12-2002

| Patent document cited in search report | | Publication date | Patent family member(s) | Publication date |
|---|---|---|---|---|
| SU 539863 | A | 25-12-1976 | SU 539863 A1 | 25-12-1976 |
| RU 2134257 | C | 10-08-1999 | RU 2134257 C1 | 10-08-1999 |